# EUROPEAN PATENT SPECIFICATION

(11) **EP 3 890 509 B1**
(45) Date of publication and mention of the grant of the patent: **27.11.2024**
(21) Application number: 19893438.2
(22) Date of filing: 05.12.2019
(51) Int. Cl.: A23L 2/56, A01N 37/06, A23L 2/60, A61K 31/20, A23L 27/10, A23L 27/00, A61K 31/201

(54) **TRAUMATIC ACID COMPOSITIONS AND METHODS FOR TASTE MODULATION**
TRAUMATISCHE SÄUREZUSAMMENSETZUNGEN UND VERFAHREN ZUR GESCHMACKSMODULATION
COMPOSITIONS D'ACIDE TRAUMATIQUE ET PROCÉDÉS DE MODULATION DU GOÛT

(30) Priority: 06.12.2018 US 201862776158 P
(43) Date of publication of application: 13.10.2021
(73) Proprietor: International Flavors & Fragrances Inc., New York, NY 10019 (US)
(72) Inventor: WU, Hou, East Brunswick, NJ 08816 (US); HUBER, Michelle Eve, Cream Ridge, NJ 08514 (US); JOHN, Thumpalasseril V., Morganville, NJ 07751 (US); LI, Xiaodong, Middletown, NJ 07748 (US)
(74) Representative: Barker Brettell LLP
(86) International application number: PCT/US2019/064594
(87) International publication number: WO 2020/118005

(56) References cited:
- EP-A1- 0 599 188
- EP-A1- 2 100 604
- WO-A2-02/098413
- CN-A- 103 932 025
- CN-A- 104 585 576
- CN-A- 105 533 449
- CN-A- 108 902 584
- JP-A- 2019 065 212
- KR-A- 20140 072 595
- KR-A- 20150 118 509
- YAMAGA MASAYUKI ET AL: "Metabolism and pharmacokinetics of medium chain fatty acids after oral administration of royal jelly to healthy subjects", RSC ADVANCES, vol. 9, no. 27, 17 May 2019 (2019-05-17), pages 15392 - 15401, XP055918584, DOI: 10.1039/C9RA02991E
- ISIDOROV ET AL.: "Royal jelly aliphatic acids contribute to antimicrobial activity of honey", JOURNAL OF APICULTURAL SCIENCE, vol. 62, no. 1, 21 June 2018 (2018-06-21), XP055715028, Retrieved from the Internet <URL:https://content.sciendo.com/configurable/contentpage/joumals$002fjas$002f62$002f1$002farticle-p111.xml> [retrieved on 20200130]

## Description

### Background

Traumatic acid is an oxidative derivative of unsaturated fatty acids and is quite common in mesophyll and meristem tissues in many plant species. Traumatic acid and its aldehyde derivative, traumatin (2-dodeceno-1-al-10-carboxylic acid), are called wound hormones, because they appear in relatively high amount around wounds and stimulate cell division.
KR 2015 0118509 A discloses a ramie cold broth and a method for producing the same.
KR 2014 0072595 A discloses natural seasoning containing ramie leaf extract and a method for preparing same for a technical field related to seasoning.
CN 103 932 025 B discloses a preparation method of Hakka ramie leaf rice.
CN 105 533 449 A discloses corn popcorn.
CN 108 902 584 A relates to the field of vegetable beverage, in particular to a vegetable protein beverage and a preparation method thereof.
JP 2019 065212 A discloses that 7-dodecenoic acid, 8-dodecenoic acid, 9-dodecenoic acid or 10-dodecenoic acid themselves have unique flavor property such as green-like, cucumber-like, oil and fat feeling and soap-like, and aroma and flavor such as milk flavor and oil and fat feeling are improved and persistence of flavor is enhanced by adding them to a flavor composition or drink and food or perfumery.

### Summary of the Invention

This invention provides a method for enhancing the sweetness of a consumable according to the claims. That is, the present invention provides a method of enhancing the sweetness of a consumable including a carbohydrate sweetener or flavoring comprising adding traumatic acid, 3-dodecenedioic acid, or combination thereof to the consumable in an amount effective to enhance the sweetness or mouthfeel of the consumable; wherein the amount of traumatic acid, 3-dodecenedioic acid, or combination thereof is at least 0.05 ppm. In certain embodiments, the method further includes the use of sebacic acid.

### Detailed Description of the Invention

It has now been found that traumatic acid and its isomer, 3-dodecenedioic acid, exhibit a sweet enhancement effect. Accordingly, the present invention provides methods which use traumatic acid and/or 3-dodecenedioic acid as an additive to enhance the sweetness of a consumable.

As is known in the art, traumatic acid (CAS No. 6402-36-4) and 3-dodecenedioic acid (CAS No. 189034-80-8) are linear, monounsaturated dicarboxylic acids. Traumatic acid and 3-dodecenedioic acid of use in this invention may be obtained from a natural source or prepared by synthetic methods. Natural sources of traumatic acid and 3-dodecenedioic acid include *Phaseolus vulgaris* and *Boehmeria nivea* leaves, as well as the leaves, stems, fruit and roots of numerous other plants, in particular wounded plant material. Traumatic acid and 3-dodecenedioic acid may be isolated from one or more of these natural sources by conventional methods. Chromatographic fractionation typically includes column chromatography and may based on molecular sizing, charge, solubility and/or polarity. Depending on the type of chromatographic method, column chromatography can be carried out with matrix materials composed of, for example, dextran, agarose, polyacrylamide or silica and can include solvents such as dimethyl sulfoxide, pyridine, water, dimethylformamide, methanol, saline, ethylene dichloride, chloroform, propanol, ethanol, isobutanol, formamide, methylene dichloride, butanol, acetonitrile, isopropanol, tetrahydrofuran, dioxane, chloroform/dichloromethane, etc. Typically, the product of the chromatographic step is collected in multiple fractions, which may then be tested for the presence of the desired compound using any suitable analytical technique (*e.g*., thin layer chromatography, mass spectrometry). Fractions enriched in the desired compound may then be selected for further purification.

Alternatively, traumatic acid or 3-dodecenedioic acid may be synthesized. For example, traumatic acid can be synthesized by converting undecylenic acid to the half-aldehyde of sebacic acid, which they then condensed with malonic acid in the presence of pyridine. CO₂ is subsequently split off after the condensation, and the unsaturated diacid is isolated after hydrolysis of the ester group (English, et al. (1941) J. Am. Chem. Soc. 63(4):941-943). See also Schreurs, et al. ((1971) Recueil des Travaux Chimiques des Pays-Bas 90(12):1331-2) for an alternative method of synthesizing traumatic acid. In yet other embodiments, traumatic acid and 3-dodecenedioic acid are obtained from a commercial source such as Cayman Chemical (Ann Arbor, MI) or Sigma-Aldrich (St. Louis, MO). In particular embodiments, the traumatic acid is purified to at least 95%, 96%, 97%, 98%, 99% or 100% homogeneity.

The compounds used in the method of the invention may have a number of isomers such as positional isomers. Accordingly, the compounds described herein include isomeric mixtures as well as single isomers that may be separated using techniques known in the art. Suitable techniques include, *e.g.,* chromatography such as high-performance liquid chromatography, referred to as HPLC, and particularly silica gel chromatography and gas chromatography trapping known as GC trapping.

While salts of traumatic acid and 3-dodecenedioic acid may be used in this invention (see, *e.g.,* US 5,567,716), preferably the traumatic acid and 3-dodecenedioic acid are not derivatized or conjugated to another molecule, *i.e*., the traumatic acid or 3-dodecenedioic acid is unconjugated traumatic acid or unconjugated 3-dodecenedioic acid.

As described herein, traumatic acid and 3-dodecenedioic acid enhance the taste and flavor of consumables. The taste and flavor profile of a consumable including traumatic acid and/or 3-dodecenedioic acid may be enhanced or more intense (*e.g.*, 5%, 10%, 20%, 30%, 40% or 50% more intense) than a comparative taste and flavor profile of a comparative consumable which does not include the traumatic acid or 3-dodecenedioic acid as an additive. Further, the mouthfeel, *e.g*., sweet and/or fatty mouthfeel, of a consumable including traumatic acid and/or 3-dodecenedioic acid may be improved in relation to the mouthfeel of a comparative consumable that does not include traumatic acid or 3-dodecenedioic acid.

In particular embodiments, traumatic acid and/or 3-dodecenedioic acid enhances the sweetness of a consumable containing a carbohydrate sweetener or flavoring. As used herein, the term "sweetness" or "sweetness intensity" refers to the relative strength of sweet sensation as observed or experienced by an individual, *e.g*., a human, or a degree or amount of sweetness detected by a taster, for example on the scale from 0 (none) to 8 (very strong) used in sensory evaluations according to the procedure described in American Society for Testing Materials, Special Technical Publication-434: "Manual on Sensory Testing Methods," ASTM International, West Conshohocken, PA (1996). The mouthfeel of a substance relates to the physical sensations in the mouth produced by a particular food. By way of illustration, a "sugary mouthfeel" is the physical sensation observed or experienced by an individual, *e.g.*, a human, upon consumption of a sugar.

As used herein, a consumable includes all food products, pharmaceutical compositions, dietary supplements, nutraceuticals, dental hygienic compositions, tabletop sweeteners, beverages, or cosmetic products. In some embodiments, the consumable includes one or more carbohydrate sweeteners or flavorings. The carbohydrate sweetener or flavoring can be present in the consumable inherently (*e.g*., in food products containing fruits) or the carbohydrate sweetener or flavoring is added into the consumable (*i.e.,* an exogenous sweetener or flavoring). Suitable carbohydrate sweeteners of the present invention include, but are not limited to, sucrose, fructose, glucose, high fructose corn syrup (containing fructose and glucose), xylose, arabinose, rhamnose, and sugar alcohols, such as erythritol, xylitol, mannitol, sorbitol, or inositol. In one embodiment, the carbohydrate sweetener is sucrose, fructose, glucose, high fructose corn syrup, xylose, arabinose or rhamnose, preferably sucrose, fructose, or glucose. In one aspect of this embodiment, the carbohydrate sweetener is sucrose. In another aspect of this embodiment, the carbohydrate sweetener is glucose. In another aspect of this embodiment, the carbohydrate sweetener is fructose. In another embodiment, the carbohydrate sweetener is a sugar alcohol, *e.g*., erythritol, xylitol, mannitol, sorbitol, or inositol.

Flavorings of use in this invention include, but are not limited to, Natural Sweet Flavor #2 (WO 2012/129451), stevioside, rebaudioside A, rebaudioside B, rebaudioside C, rebaudioside D, rebaudioside E, rebaudioside F, dulcoside A, dulcoside B, stevia, alpha-glucosyl stevia, fructosyl stevia, galactosyl stevia, beta-glucosyl stevia, siamenoside, mogroside IV, mogroside V, Luo Han Guo sweetener, monatin and its salts, glycyrrhizic acid and its salts (*e.g*., as found in MAGNASWEET), curculin, thaumatin, monellin, mabinlin, brazzein, hernandulcin, phyllodulcin, glycyphyllin, phloridzin, trilobtain, baiyunoside, osladin, polypodoside A, pterocaryoside A, pterocaryoside B, mukurozioside, phlomisoside I, periandrin I, abrusoside A, cyclocarioside I, or a combination thereof. In certain embodiments, the flavoring is Natural Sweet Flavor #2 (also known as NSF-02), which contains glucosylated steviol glycosides and dextrin.

When added to a consumable as an additive, traumatic acid and/or 3-dodecenedioic acid is used in an amount effective to enhance the sweetness of a carbohydrate sweetener or flavoring without exhibiting any off-taste. Any amount of traumatic acid and/or 3-dodecenedioic acid that provides the desired degree of sweetness enhancement can be used. Preferably, the amount of traumatic acid and/or 3-dodecenedioic acid present in the consumable is an amount as low as 0.05 ppm, in an amount as low as 0.2 ppm, in an amount as low as 1 ppm, or in an amount as low as 10 ppm. The traumatic acid can be included in the consumable in an amount that is as high as 1000 ppm, in an amount as high as 500 ppm, or in an amount as high as 100 ppm. The traumatic acid may further be present within any range delimited by any pair of the foregoing values, such as between 0.2 ppm and 1000 ppm, or between 1 ppm and 100 ppm, for example. The term "ppm" as used herein means part per million by weight or volume, for example, the weight of the component (in milligrams) per liter of solution, *i.e*., µg/ml.

In some embodiments, the invention embraces a combination of traumatic acid and/or 3-dodecenedioic acid with sebacic acid (CAS No. 111-20-6). In particular embodiments, the weight ratio of traumatic acid and/or 3-dodecenedioic acid to sebacic acid is at least 1:1, 2:1, 3:1, 4:1, 5:1, 6:1, 7:1, 8:1, or 9:1.

The phrase "food product" as used herein includes, but is not limited to, fruits, vegetables, juices, meat products (*e.g*., ham, bacon and sausage), egg products, fruit concentrates, gelatins and gelatin-like products (*e.g.,* jams, jellies, preserves, and the like) milk products (*e.g*., ice cream, sour cream and sherbet), icings, syrups including molasses, corn products, wheat products, rye products, soybean products, oat products, rice products and barley products, nut meats and nut products, cakes, cookies, confectionaries (*e.g.,* candies, gums, fruit flavored drops, and chocolates), chewing gum, mints, creams, ice cream, pies and breads, and beverages such as coffee, tea, carbonated soft drinks (*e.g*., soft drinks sold under the tradenames COKE^{®} and PEPSI^{®}), non-carbonated soft drinks, juices and other fruit drinks, sports drinks such as those sold under the tradename GATORADE^{®}, alcoholic beverages, such as beers, wines and liquors, and flavored drinks sold under the tradename KOOL-AID^{®}. Food products also include condiments such as herbs, spices and seasonings, and flavor enhancers, such as monosodium glutamate. A food product also includes prepared packaged products, such as dietetic sweeteners, liquid sweeteners, granulated flavor mixes which upon reconstitution with water provide non-carbonated drinks, instant pudding mixes, instant coffee and tea, coffee whiteners, malted milk mixes, pet foods, livestock feed, tobacco, and materials for baking applications, such as powdered baking mixes for the preparation of breads, cookies, cakes, pancakes, donuts and the like. Food products also include diet or low-calorie food and beverages containing little or no sucrose. Especially preferred food products are carbonated beverages. Preferably, the consumable in which the sweetness is enhanced contains a decreased level of the carbohydrate sweetener. For example, an improved carbonated soft drink can be produced with the same sweetness as the known carbonated soft drink but with a lower sugar content by adding traumatic acid and/or 3-dodecenedioic acid.

The consumable can also be a pharmaceutical composition. Preferred compositions are pharmaceutical compositions containing traumatic acid and one or more pharmaceutically acceptable excipients. These pharmaceutical compositions can be used to formulate pharmaceutical drugs containing one or more active agents that exert a biological effect other than sweetness enhancement. The pharmaceutical composition preferably further includes one or more active agents that exert a biological effect. Such active agents include pharmaceutical and biological agents that have an activity other than taste enhancement. Such active agents are well known in the art. See, e.g., The Physician's Desk Reference. Such compositions can be prepared according to procedures known in the art, for example, as described in Remington's Pharmaceutical Sciences, Mack Publishing Co., Easton, PA. In one embodiment, such an active agent includes bronchodilators, anorexiants, antihistamines, nutritional supplements, laxatives, analgesics, anesthetics, antacids, H₂-receptor antagonists, anticholinergics, antidiarrheals, demulcents, antitussives, antinauseants, antimicrobials, antibacterials, antifungals, antivirals, expectorants, anti-inflammatory agents, antipyretics, and mixtures thereof. In one embodiment, the active agent is a antipyretic or analgesic, *e.g.,* ibuprofen, acetaminophen, or aspirin; laxative, *e.g.,* phenolphthalein dioctyl sodium sulfosuccinate; appetite depressant, *e.g.,* amphetamine, phenylpropanolamine, phenylpropanolamine hydrochloride, or caffeine; antacidic, *e.g.,* calcium carbonate; antiasthmatic, *e.g.,* theophylline; antidiuretic, *e.g.,* diphenoxylate hydrochloride; agent active against flatulence, *e.g.,* simethecon; migraine agent, *e.g.,* ergotaminetartrate; psychopharmacological agent, *e.g.,* haloperidol; spasmolytic or sedative, *e.g.,* phenobarbitol; antihyperkinetic, *e.g.,* methyldopa or methylphenidate; tranquilizer, *e.g.,* a benzodiazepine, hydroxinmeprobramate or phenothiazine; antihistaminic, *e.g.,* astemizol, chloropheniramine maleate, pyridamine maleate, doxlamine succinate, bromopheniramine maleate, phenyltoloxamine citrate, chlorocyclizine hydrochloride, pheniramine maleate, or phenindamine tartrate; decongestant, *e.g.,* phenylpropanolamine hydrochloride, phenylephrine hydrochloride, pseudoephedrine hydrochloride, pseudoephedrine sulfate, phenylpropanolamine bitartrate, or ephedrine; beta-receptor blocker, *e.g.,* propanolol; agent for alcohol withdrawal, *e.g.,* disulfuram; antitussive, *e.g.,* benzocaine, dextromethorphan, dextromethorphan hydrobromide, noscapine, carbetapentane citrate, or chlophedianol hydrochloride; fluorine supplement, *e.g.,* sodium fluoride; local antibiotic, *e.g.,* tetracycline or cleocine; corticosteroid supplement, *e.g.,* prednisone or prednisolone; agent against goiter formation, *e.g.,* colchicine or allopurinol; antiepileptic, *e.g.,* phenyloine sodium; agent against dehydration, *e.g.,* electrolyte supplement; antiseptic, *e.g.,* cetylpyridinium chloride; NSAID, *e.g.,* acetaminophen, ibuprofen, naproxen, or salt thereof; gastrointestinal active agent, *e.g.,* loperamide and famotidine; an alkaloid, *e.g.,* codeine phosphate, codeine sulfate, or morphine; supplement for a trace element, *e.g.,* sodium chloride, zinc chloride, calcium carbonate, magnesium oxide, or other alkali metal salt or alkali earth metal salt; vitamin; ion-exchange resin, *e.g.,* cholestyramine; cholesterol-depressant or lipid-lowering substance; antiarrhythmic, *e.g.,* N-acetylprocainamide; or expectorant, *e.g.,* guaifenesin.

In some embodiments, the consumable is a dietary supplement or nutraceutical. Examples of such compositions having an undesirable taste include, but are not limited to, enteral nutrition products for treatment of nutritional deficit, trauma, surgery, Crohn's disease, renal disease, hypertension, obesity and the like, to promote athletic performance, muscle enhancement or general well-being or inborn errors of metabolism such as phenylketonuria. In particular, such compositions can contain one or more amino acids which have a bitter or metallic taste or aftertaste. Such amino acids include, but are not limited to, essential amino acids such as L isomers of leucine, isoleucine, histidine, lysine, methionine, phenylalanine, threonine, tryptophan, tyrosine, and valine.

In a further embodiment, the consumable of the present invention is a dental hygienic composition, containing a carbohydrate sweetener or flavoring and traumatic acid and/or 3-dodecenedioic acid. Dental hygienic compositions are known in the art and include, but are not necessarily limited to, toothpaste, mouthwash, plaque rinse, dental floss, dental pain relievers (such as ANBESOL^{™}), and the like. In one embodiment, the dental hygienic composition includes one carbohydrate sweetener. In another embodiment, the dental hygienic composition includes more than one carbohydrate sweetener. In certain embodiments, the dental hygienic composition includes sucrose and corn syrup, or sucrose and aspartame.

In yet another embodiment, the consumable of the present invention is a cosmetic product containing a carbohydrate sweetener or flavoring and traumatic acid and/or 3-dodecenedioic acid. For example, but not by way of limitation, the cosmetic product can be a face cream, lipstick, lip gloss, and the like. Other suitable compositions of the invention include lip balm, such as lip balm sold under the tradename CHAPSTICK^{®} or BURT'S BEESWAX^{®}.

When used in the methods of this invention, traumatic acid and/or 3-dodecenedioic acid is added to a consumable (*i.e*., the traumatic acid and/or 3-dodecenedioic acid is not an endogenous component of the consumable) in an amount effective to enhance the sweetness of the consumable including a carbohydrate sweetener or flavoring. In this respect, the amount of carbohydrate sweetener or flavoring added to the consumable may be reduced while retaining the desired sweetness level. Thus, the present invention also provides methods for enhancing the sweetness of a carbohydrate sweetener and/or flavoring and decreasing the amount of a carbohydrate sweetener and/or flavoring in a consumable by combining the carbohydrate sweetener and/or flavoring with traumatic acid and/or 3-dodecenedioic acid. In some embodiments, the weight ratio of carbohydrate sweetener or flavoring to traumatic acid and/or 3-dodecenedioic acid is 10000:1, 1000:1, or 100:1. The disclosure provides, but does not claim, a consumable containing an effective amount of a combination of (a) traumatic acid and/or 3-dodecenedioic acid and (b) a carbohydrate sweetener or flavoring in a reduced amount in order to achieve the same level of sweetness when the carbohydrate sweetener or flavoring is used alone in the traditional amount. In this respect, the amount of carbohydrate sweetener or flavoring used in a consumable can be reduced by at least about 5%, 10%, 20%, 30% or more. Unless otherwise specified, percentages (%) are by weight.

The invention is described in greater detail by the following non-limiting examples.

### Example 1: Sensory Evaluation of Traumatic Acid at Different Amounts in Sugar Water

Sugar water (4% sucrose in drinking water) was prepared and used as a base solution. Different amounts of traumatic acid were added to the base solution and descriptive sensory evaluations were performed (Table 1).

**TABLE 1**

| Traumatic acid (ppm) | Taste Evaluation |
|---|---|
| 0.02 | Slight sweet and mouthfeel enhancement, barely detectable. |
| 1000 | Strong sweet and mouthfeel enhancement with strong chemical off-taste |

### Example 2: Sensory Evaluation of Traumatic acid and 3-Dodecenedioic Acid in Sugar Water

Sugar water (4% sucrose in drinking water) was prepared and used as a base solution. Traumatic acid and 3-dodecenedioic acid were added to the base solution and descriptive sensory evaluations were performed (Table 2).

**TABLE 2**

| Compound (5 ppm) | Taste Evaluation |
|---|---|
| Traumatic acid | Sweeter than the base solution, mouthcoating, fatty sensation |
| 3-Dodecenedioic acid | Sweeter than the base solution, fruity, fatty sensation |

The results of this analysis indicated that both traumatic acid and 3-dodecenedioic acid enhanced the sweetness and mouthfeel of sugar water.

### Example 3: Taste Modulation Using Traumatic Acid

Descriptive sensory evaluations were carried out using different amounts of traumatic acid in different applications. The results of this analysis, as compared to the same applications in the absence of traumatic acid, are presented in Table 3.

**TABLE 3**

| Application | Traumatic Acid | Taste Evaluation |
|---|---|---|
| Water | 40 ppm | Fatty and oily |
| 3% Sugar + 0.05% Citric Acid Water Solution | 40 ppm | Fatty and oily |
| 3% Sugar - Sweetened Full Fat Milk | 7.5 ppm | Enhances fatty perception and creaminess, makes similar to sweet cream |
| Strawberry Flavored Yogurt (5% Sugar, 0.4% Fat, 8.7 Grams Protein) | 15 ppm | Sweet up front, more strawberry |
| Mixed Berry Yogurt Smoothie (1% Fat, 9% Sugar, 3% Protein) | 7.5 ppm | More sweet creamy, milkier than control, more indulgent |

### Example 4: Sensory Evaluation of Traumatic Acid and Sebacic Acid in Sweetened Milk

Sweetened milk (2% fat milk with 2% sucrose) was prepared and used as a base solution ("base"). Sebacic acid and traumatic acid were added to the base solution and descriptive sensory evaluations were performed (Table 4).

**TABLE 4**

| Sample (ppm) | Taste Evaluation | Strength |
|---|---|---|
| Base (N/A) | Sweetened milk taste | + |
| Traumatic Acid (2 ppm) | Sweeter and more mouthfeel than the base, slightly coconutty, and waxy. More overall dairy impression and more dairy fat aroma | +++ |
| Sebacic Acid (2 ppm) | Sweeter and more mouthfeel than the base. More overall dairy impression and more dairy fat aroma | ++ |
| Traumatic Acid (2 ppm) and Sebacic Acid (2 ppm) | Sweeter and more mouthfeel than the base. More overall dairy impression and more dairy fat aroma | ++++ |

The results of this analysis indicated that traumatic acid (2 ppm) and sebacic acid (2 ppm) each increased sweetness and mouthfeel of sweetened milk. When used together, traumatic acid (2 ppm) and sebacic acid (2 ppm) provided the most increase in sweetness and mouthfeel.

In addition, the sweetness and mouthfeel enhancement as well as off-flavor masking of traumatic acid was compared with a series of analogs including mesaconic acid, glutaconic acid, trans-β-Hydromuconic acid, and e-oct-4ene-1, 8-dioic acid at 5 ppm. Among all the compounds tested, traumatic acid exhibited the strongest and the longest-lasting effect, which was significantly superior to all other compounds.

## Claims

1. A method of enhancing the sweetness of a consumable including a carbohydrate sweetener or flavoring comprising adding traumatic acid, 3-dodecenedioic acid, or combination thereof to the consumable in an amount effective to enhance the sweetness of the consumable;
wherein the amount of traumatic acid, 3-dodecenedioic acid, or combination thereof is at least 0.05 ppm.

2. The method of claim 1, further comprising adding sebacic acid to the consumable.

3. The method of claim 1 or of claim 2, wherein the amount of traumatic acid, 3-dodecenedioic acid, or combination thereof is in the range of 0.2 ppm to 1000 ppm.

4. The method of claim 1 or of claim 2, wherein the amount of traumatic acid, 3-dodecenedioic acid, or combination thereof is in the range of 1 ppm to 100 ppm.

5. The method of claim 1 or of any of claims 2 to 4, wherein the consumable is a food product, pharmaceutical composition, a dietary supplement, a nutraceutical, a dental hygienic composition, a tabletop sweetener, a beverage, or a cosmetic product.

## Patentansprüche

1. Verfahren zur Verstärkung der Süße eines Verbrauchsguts, das ein Kohlenhydrat-Süßungsmittel oder einen Kohlenhydrat-Geschmacksstoff enthält, umfassend das Zugeben von Traumatinsäure, 3-Dodecendisäure oder einer Kombination davon zu dem Verbrauchsgut in einer zur Verstärkung der Süße des Verbrauchsguts wirksamen Menge;
wobei die Menge an Traumatinsäure, 3-Dodecendisäure oder einer Kombination davon mindestens 0,05 ppm beträgt.

2. Verfahren nach Anspruch 1, ferner umfassend das Zugeben von Sebacinsäure zu dem Verbrauchsgut.

3. Verfahren nach Anspruch 1 oder Anspruch 2, wobei die Menge an Traumatinsäure, 3-Dodecendisäure oder einer Kombination davon im Bereich von 0,2 ppm bis 1000 ppm liegt.

4. Verfahren nach Anspruch 1 oder Anspruch 2, wobei die Menge an Traumatinsäure, 3-Dodecendisäure oder einer Kombination davon im Bereich von 1 ppm bis 100 ppm liegt.

5. Verfahren nach Anspruch 1 oder einem der Ansprüche 2 bis 4, wobei es sich bei dem Verbrauchsgut um ein Lebensmittelprodukt, eine pharmazeutische Zusammensetzung, eine Nahrungsergänzung, ein Nutrazeutikum, eine Zahnhygienezusammensetzung, ein Tafelsüßungsmittel, ein Getränk oder ein Kosmetikprodukt handelt.

## Revendications

1. Méthode destinée à améliorer la sucrosité d'un produit consommable comportant un aromatisant ou un édulcorant à base de glucide, comprenant l'addition d'acide traumatique, d'acide 3-dodécènedioïque, ou d'une combinaison de ceux-ci au produit consommable selon une quantité efficace pour améliorer la sucrosité du produit consommable ;
dans laquelle la quantité d'acide traumatique, d'acide 3-dodécènedioïque, ou d'une combinaison de ceux-ci est d'au moins 0,05 ppm.

2. Méthode selon la revendication 1, comprenant en outre l'addition d'acide sébacique au produit consommable.

3. Méthode selon la revendication 1 ou la revendication 2, dans laquelle la quantité d'acide traumatique, d'acide 3-dodécènedioïque, ou d'une combinaison de ceux-ci se trouve dans la plage allant de 0,2 ppm à 1000 ppm.

4. Méthode selon la revendication 1 ou la revendication 2, dans laquelle la quantité d'acide traumatique, d'acide 3-dodécènedioïque, ou d'une combinaison de ceux-ci se trouve dans la plage allant de 1 ppm à 100 ppm.

5. Méthode selon la revendication 1 ou l'une quelconque des revendications 2 à 4, dans laquelle le produit consommable est un produit alimentaire, une composition pharmaceutique, un complément alimentaire, un produit nutraceutique, une composition d'hygiène dentaire, un édulcorant de table, une boisson ou un produit cosmétique.
